# EUROPÄISCHE PATENTSCHRIFT

(11) **EP 4 032 095 B1**
(45) Veröffentlichungstag und Bekanntmachung des Hinweises auf die Patenterteilung: **30.04.2025**
(21) Anmeldenummer: 20774972.2
(22) Anmeldetag: 17.09.2020
(51) Int. Cl.: G16H 10/60, G16H 40/20, G16H 40/63, G16H 40/67

(54) **MEDIZINISCHE PATIENTENVALIDIERUNGSEINRICHTUNG**
MEDICAL PATIENT VALIDATION DEVICE
SYSTÈME MÉDICAL DE VALIDATION DE PATIENT

(30) Priorität: 17.09.2019 DE 102019124995
(43) Veröffentlichungstag der Anmeldung: 27.07.2022
(73) Patentinhaber: B. Braun Melsungen AG, 34212 Melsungen (DE)
(72) Erfinder: SCHMOLL, Horst, 34302 Guxhagen (DE)
(74) Vertreter: Winter, Brandl - Partnerschaft mbB
(86) Internationale Anmeldenummer: PCT/EP2020/076003
(87) Internationale Veröffentlichungsnummer: WO 2021/053082

(56) Entgegenhaltungen:
- US-A1- 2005 102 167

## Beschreibung

### Technisches Gebiet

Die vorliegende Offenbarung betrifft ein Validierungssystem (-einrichtung) und ein Kontrollverfahren zur Sicherstellung, dass zumindest eine Patienten- und/oder Standortinformation sicher einem medizinischen Gerät zugeordnet und die Zuordnung überprüft wird.

### Hintergrund der Erfindung

In der klinischen Praxis sind medizinische Geräte mit Patienten verbunden. Sowohl das medizinische Gerät als auch der Patient werden mittels eines "Readers" identifiziert und das dabei ausgelesene Datenpaket wird an einen Server gesendet. Die ausgelesenen Daten enthalten insbesondere Patienten- und/oder Standortinformationen, welche zumindest einem medizinischen Gerät zugeordnet sind. Die Standortinformationen sind vorzugsweise Informationen zu dem Standort des Patientenbetts oder Patientenzimmers sowie der medizinischen Geräte, welche sich meist dem Patientenzimmer bzw. in der Nähe des Patientenbettes befinden.

Es ist ein Datentransfer zwischen dem Server und zumindest einem medizinischen Gerät, wie beispielsweise eine oder mehrere Infusionspumpen, vorgesehen. Hierbei kann das medizinische Geräte beispielsweise ein Rezept und/oder eine Medikation für einen bestimmten Patienten empfangen. Bei diesem Datentransfer ist es wichtig, dass die von dem medizinischen Gerät zu empfangenden Daten an den richtigen Patienten gelangen.

Es sind Verfahren zur logischen Zuordnung von Medizinprodukten zu Patienten bereits bekannt. Diese dienen beispielsweise zur automatischen Programmierung von Infusionspumpen. In aktuellen Implementierungen, bei welchen Medizinprodukte dem Patienten logisch zugeordnet werden, sind im Hinblick auf die Norm IEC 60601-1 nicht einfach fehlersicher. In der klinischen Praxis bedeutet dies, dass die Zuordnungsinformationen nicht vollständig vertrauenswürdig sind und eine zusätzliche Validierung des Benutzers erfordern. So müssen beispielsweise Therapieverordnungen, die an eine Infusionspumpe gesendet werden, vor Beginn der Infusion vom Benutzer validiert und bestätigt werden.

### Stand der Technik

Nach dem aktuellen Stand der Technik weiß der Server, welche Patienten-ID zu einem bestimmten Medizinprodukt gehört. Während der Server in der Lage ist, Informationen für diese Patienten-ID an das Medizinprodukt weiterzuleiten, ist das Verfahren nicht ausfallsicher. Ein Fehler, wie er in den bisherigen Systemen auftreten kann, kann gemäß diesem Stand der Technik nicht erkannt werden und kann zu Fehlleitungen führen.

In anderen Worten ausgedrückt ermöglichen es aktuelle Implementierungen, Medizinprodukte nur den Patienten logisch zuzuordnen. Eine Validierung oder Verifizierung dieser Zuordnung ist jedoch nicht vorgesehen. Dabei ist es ferner nachteilig, dass Standorte wie ein Zimmer oder eine Patientenbetten-ID nicht abgedeckt sind.

US 2005/102167 A1 offenbart ein Validierungssystem, mit zumindest einem medizinischen Gerät, welches an einen Patienten angeschlossen ist und sowohl das medizinische Gerät als auch der Patient und/oder ein Patientenbett mit jeweils zumindest einer Identifizierungsmarkierung versehen ist.

### Kurzbeschreibung der Erfindung

Der vorliegenden Erfindung liegt daher die Aufgabe zugrunde, ein Validierungssystem für die Zuordnung von zumindest einem medizinischen Gerät zu einem Patienten, welches die Nachteile aus dem Stand der Technik vermeidet oder zumindest verringert. Die vorliegende Erfindung wird durch die beigefügten Ansprüche definiert.

Der Kern der vorliegenden Erfindung liegt darin, ein Validierungssystem mit zumindest einem medizinischen Gerät, einer Identifizierungsvorrichtung, und einem Server bereitzustellen, der mit dem zumindest einen medizinischen Gerät und der Identifizierungsvorrichtung kommuniziert und dazu vorgesehen und ausgebildet ist, das zumindest eine medizinische Gerät einem entsprechenden Patienten und/oder Patientenbett bzw. Patienteninformationen und/oder Standortinformationen gemäß einem Zuordnungsdurchlauf zuzuordnen. Das zumindest eine medizinische Instrument ist mit einem Patienten verbunden bzw. verbindbar und sowohl das medizinische Gerät als auch der Patient ist jeweils mit zumindest einer Identifikationsmarkierung versehen bzw. versehbar. Die Identifizierungsvorrichtung ist mit einem Display versehen und ist dazu vorgesehen und ausgebildet, die Identifikationsmarkierungen auszulesen.

Erfindungsgemäß ist zusätzlich ein Überprüfungsmittel angeordnet, das dazu vorgesehen und ausgebildet ist, in einem zu dem Zuordnungsdurchlauf unabhängigen/separaten Überprüfungsdurchlauf mittels einer von dem Server generierten ersten Transaktions-ID die Zuordnung des zumindest einen medizinischen Geräts zu dem entsprechenden Patienten und/oder Patientenbett bzw. die entsprechenden Patienteninformationen und/oder Standortinformationen zu überprüfen.

In anderen Worten bedeutet das, dass der Patient und/oder das Patientenbett und das zumindest eine medizinische Gerät jeweils mit einer Identifikationsmarkierung versehen sind, welche gemäß einem Zuordnungsdurchlauf mittels der Identifizierungsvorrichtung ausgelesen werden. Die ausgelesenen Daten aus der Identifizierungsmarkierung von dem Patienten und/oder dem Patientenbett und dem zumindest einen entsprechenden medizinischen Gerät werden als Datenpaket an den Server gesendet. In einem Überprüfungsdurchlauf wird anschließend auf Basis des vom Server empfangenen Datenpakets eine erste Transaktions-ID generiert. Die generierte erste Transaktions-ID ermöglicht es, die Zuordnung des zumindest einen medizinischen Geräts zu dem entsprechenden Patienten und/oder Patientenbett bzw. Patienteninformation und/oder Standortinformationen zu überprüfen.

Das vorstehend beschriebene Validierungssystem bietet die Grundlage für medizinische Serveranwendungen, die Daten mehrerer medizinischer Geräte/Medizinprodukte verwenden, die mit einem einzigen Patienten und/oder Standort verknüpft sind/in Verbindung stehen.

Da es ferner bevorzugt ist, wenn die Identifizierungsvorrichtung als eine Verbindungsvorrichtung ausgebildet ist, ermöglicht das Validierungssystem eine Verbindung zwischen dem Server bzw. der Serveradresse und dem medizinische Gerät und/oder Patient und/oder Patientenbett und/oder angeschlossenen Systemen, um von dem medizinische Gerät an den Server übermittelte Daten sicher dem Patienten und/oder dem Patientenbett zuzuordnen. Dies ermöglicht einem Anwender, insbesondere einem Arzt, das Erstellen einer Diagnose und/oder einer Anpassung der Behandlung. Insbesondere ist es bevorzugt, wenn zudem die Verbindung zwischen dem Server bzw. der Serveradresse und dem medizinischen Gerät und/oder dem Patient und/oder dem Patientenbett mittels einer Fernsteuerung hergestellt und genutzt wird. Es ist bevorzugt, wenn die Identifizierungsvorrichtung die Identifikationsmarkierungen optisch, zum Beispiel mittels Infrarot, ausliest. Dies bietet den Vorteil, dass der Anwender/Bediener der Identifizierungsvorrichtung einen variablen Abstand und/oder Winkel zu der Identifikationsmarkierung haben kann/darf.

Dadurch ist es möglich, dynamisch eine sichere, logische Zuordnung und Verknüpfung von Medizinprodukten zu Patienten- und/oder Standortinformationen zu gewährleisten. Dabei ist es vorgesehen, dass die Patienten und/oder Standortinformationen in technischen oder medizinischen Daten enthalten sind, die von dem zumindest einen medizinischen Geräte/Medizinprodukt gemeldet werden und die Verknüpfung/Zuordnung von Daten einer Vielzahl von medizinischen Geräten/Medizinprodukten mit/zu einem Patienten und/oder Standort ermöglichen. Alle Daten, die von einer Medizinprodukteseite empfangen werden, wie beispielsweise eine Therapieverordnung für einen bestimmten Patienten, können so sicher überprüft werden, ob diese an exakt das medizinische Gerät gesendet werden, das an den richtigen Patienten und/oder Standort angeschlossen ist.

Vorteilhafte Ausgestaltungen sind in den Unteransprüchen beansprucht und werden nachstehend näher erläutert.

Es ist bevorzugt, wenn die Identifikationsmarkierung als ein QR-Code oder Barcode oder eine Identifikationsnummer ausgebildet ist und zumindest eine Geräte-ID und eine Patienten-ID und/oder eine Standort-ID aufweist. In anderen Worten bedeutet das, dass der Patient beispielsweise ein Armband mit einer entsprechenden Identifikationsmarkierung trägt, welche die Patienten-ID aufweist. Des Weiteren ist alternativ oder zusätzlich vorgesehen, dass das Patientenbett eine Identifikationsmarkierung aufweist, die die Standort-ID, vorzugsweise die Patientenbett-ID und/oder das zugehörige Zimmer, ausgibt. An dem zumindest einen medizinischen Gerät ist ebenfalls eine Identifikationsmarkierung angebracht, welche die Geräte-ID ausgibt. Dies hat den Vorteil, dass die Identifikationsmarkierung leicht mittels der Identifikationsvorrichtung ausgelesen werden können. Die Identifikationsvorrichtung ist vorzugsweise als QR-Code-Reader und/oder Barcode-Reader oder alternativ als optisches Kamerasystem ausgebildet.

Es ist bevorzugt, dass sich die von dem Server generierte erste Transaktions-ID aus der Geräte-ID und der Patienten-ID und/oder der Standort-ID zusammensetzt. Die Gesundheitsorganisation führt eine Liste von eindeutigen Identifikationselementen für Medizinprodukte, medizinische Geräte, Patienten und/oder Standorten. Dies ist beispielsweise ein Patientenarmband mit einer eindeutigen Identifikationsnummer oder einem QR-Code mit Informationen zur Identifizierung der Medizinprodukte/ medizinischen Geräten oder Standortinformationen.

Das medizinische Gerät ist in dem Überprüfungsdurchlauf dazu vorgesehen und ausgebildet, die erste Transaktions-ID zu empfangen und eine geänderte zweite Transaktions-ID an den Server zu senden, wenn die erste Transaktions-ID die Geräte-ID aufweist. In anderen Worten heißt das, dass der Server die generierte erste Transaktions-ID an das zumindest eine medizinische Gerät sendet. Das zumindest eine medizinische Gerät gleicht ab, ob in der ersten Transaktions-ID die dem zumindest einen medizinischen Gerät entsprechende Geräte-ID enthalten ist. Ist das der Fall, zeigt das zumindest eine medizinische Gerät eine geänderte/modifizierte zweite Transaktions-ID auf dem Bildschirm der Identifizierungsvorrichtung an.

Der Server ist in dem Überprüfungsdurchlauf dazu vorgesehen und ausgebildet, die empfangene zweite Transaktions-ID mit der generierten und im Server gespeicherten ersten Transaktions-ID zu vergleichen. In anderen Worten bedeutet das, dass die zweite Transaktions-ID von der Identifizierungsvorrichtung zurück an den Server gesendet wird. Der Server vergleicht die zweite Transaktions-ID mit ersten Transaktions-ID, wobei die erste Transaktions-ID im Server gespeichert ist/war.

Es ist bevorzugt, wenn bei einem positiven Vergleich in dem medizinischen Gerät, die Identifizierungsvorrichtung in dem Überprüfungsdurchlauf dazu vorgesehen und ausgebildet ist, die positive Zuordnung des medizinischen Geräts zu dem Patienten und/oder zu dem Ort in einem vorzugsweise optischen Symbol auf dem Display zu visualisieren. Das bedeutet, dass bei Übereinstimmung der ersten Transaktions-ID mit der zweiten Transaktions-ID die Zuordnung des zumindest einen medizinischen Geräts zu dem Patienten und/oder Standort als gültig behandelt wird. Die gültige Verbindung/Zuordnung des zumindest einen medizinischen Geräts/Produkts zu dem Patienten und/oder Patientenbett bzw. zu den Patienteninformationen und/oder Standortinformationen wird schließlich durch ein der Identifizierungsvorrichtung zugeordnetes optisches Symbol visualisiert.

Es ist bevorzugt, wenn der Server in dem Überprüfungsdurchlauf dazu vorgesehen und ausgebildet ist, die zweite Transaktions-ID in eine dritte Transaktions-ID zu ändern und an das medizinische Gerät zu senden. In anderen Worten heißt das, dass die vom Server empfangene zweite Transaktions-ID nochmals modifiziert wird und an das zumindest eine medizinische Gerät gesendet wird.

Es ist bevorzugt, wenn das medizinische Gerät in dem Überprüfungsdurchlauf dazu vorgesehen und ausgebildet ist, die empfangene dritte Transaktions-ID mit der gespeicherten zweiten Transaktions-ID zu vergleichen. Das zumindest eine medizinische Gerät vergleicht diese modifizierte dritte Transaktions-ID mit der in dem zumindest einen medizinischen Gerät gespeicherten zweiten Transaktions-ID.

Es ist bevorzugt, wenn bei einem positiven Vergleich, die Identifizierungsvorrichtung in dem Überprüfungsdurchlauf dazu vorgesehen und ausgebildet ist, die positive Zuordnung des zumindest einen medizinischen Geräts zu dem Patienten und/oder zu dem Ort in einem vorzugsweise optischen Symbol auf dem Display zu visualisieren. Das bedeutet, dass bei Übereinstimmung der zweiten Transaktions-ID mit der dritten Transaktions-ID die Zuordnung des zumindest einen medizinischen Geräts zu dem Patienten und/oder Patientenbett bzw. zu den Patienteninformationen und/oder Standortinformationen als gültig behandelt wird. Die gültige Verbindung/Zuordnung des zumindest einen medizinischen Geräts/Produkts zu dem Patienten und/oder Standort wird schließlich durch ein optisches Symbol auf dem Bildschirm der Identifizierungsvorrichtung visualisiert.

Es ist bevorzugt, wenn das Validierungssystem dazu vorgesehen und ausgebildet ist, die Patienten- und/oder Standortinformationen von einer Vielzahl von Medizingeräten/-produkten, welche einem einzigen Patienten oder Standort zugeordnet sind, zu gruppieren. In anderen Worten bedeutet das, dass zu diesem Zeitpunkt alle von dem zumindest einen medizinischen Geräte gesendeten Daten Patienten- und/oder Standortinformation enthalten/ausweisen, die es ermöglichen, Daten einer Vielzahl von medizinischen Geräten/Medizinprodukten zu kombinieren. Darüber hinaus ist das zumindest eine medizinische Gerät dazu ausgebildet/in der Lage, eine unabhängige Validierung von eingehenden Kontrollaufträgen durchzuführen.

Zusammenfassend ermöglicht das Validierungssystem die Überprüfung der in dem Zuordnungsdurchlauf vorgenommenen Zuordnung. Somit bietet diese Überprüfung mittels des Überprüfungsmittels einen zum Zuordnungsdurchlauf unabhängigen Kanal zum Schutz von Einzelfehlern. Somit bietet das Validierungssystem den Vorteil, eine fehlersichere Zuordnung von Daten von Medizinprodukten/medizinischen Geräten zu einer Patienten-ID und/oder einer Standort-ID und ermöglicht durch den Überprüfungsdurchlauf, Daten in Serveranwendungen für therapeutischen Entscheidungen zu verwenden, Daten einer Vielzahl von Medizinprodukten/medizinischen Geräten, die zu einem einzigen Patienten/Standort gehören, zu gruppieren und Kontrolldaten zur Fernsteuerung von Medizinprodukten/medizinischen Geräte zu übernehmen.

Des Weiteren betrifft die vorliegende Erfindung ein Kontrollverfahren zur Sicherstellung, dass zumindest eine Patienten- und/oder Standortinformation zumindest einem medizinischen Gerät zugeordnet und die Zuordnung überprüft wird, mit den nachfolgenden Schritten.

In einem ersten Schritt erfolgen das Auslesen einer Identifikationsmarkierung und das Ausgeben einer Geräte-ID, Patienten-ID und/oder Standort-ID als ein Datenpaket durch eine Identifizierungsvorrichtung. Diese ausgelesenen Daten werden in einem zweiten Schritt als Datenpaket an einen Server gesendet. Der Server generiert eine erste Transaktions-ID aus der Geräte-ID, Patienten-ID und/oder Standort-ID und sendet die generierte erste Transaktions-ID an das zumindest eine medizinische Gerät. In einem weiteren Schritte wird die erste Transaktions-ID in eine zweite Transaktions-ID geändert/modifiziert, wenn die erste Transaktions-ID die Geräte-ID aufweist. Das bedeutet, dass das zumindest eine medizinische Gerät prüft, ob dessen Geräte-ID in der ersten Transaktions-ID enthalten ist. Sofern dies der Fall ist, wird die erste Transaktions-ID modifiziert und auf dem Display der Identifizierungsvorrichtung angezeigt.

In einem darauffolgenden Schritt wird die zweite Transaktions-ID an den Server zurückgesendet und die am Server empfangene zweite Transaktions-ID mit der im Server gespeicherten ersten Transaktions-ID verglichen. Bei einem übereinstimmenden Vergleich wird ein optisches Symbol auf dem Display der Identifizierungsvorrichtung visualisiert und die zweite Transaktions-ID in eine dritte Transaktions-ID in dem Server geändert.

Die dritte Transaktions-ID wird an das zumindest eine medizinische Gerät gesendet und die empfangene dritte Transaktions-ID mit der gespeicherten zweiten Transaktions-ID verglichen. In einem letzten Schritt wird ein optisches Symbol auf dem Bildschirm der Identifizierungsvorrichtung bei übereinstimmendem Vergleich visualisiert und angezeigt.

Nach erfolgreicher Durchführung der vorstehenden Schritte wird die logische Verknüpfung/Zuordnung von Medizinprodukt/medizinisches Gerät zu Patient und/oder Standort sowohl server- als auch medizingeräteseitig validiert.

Durch Wiederholen dieses Verfahrens, kann eine Vielzahl von mehreren/verschiedenen medizinischen Geräten/Medizinprodukten, wie beispielsweise mehrere Infusionspumpen, Dialysegeräte, Beatmungsgeräte, physiologische Sensoren und/oder Ähnliches, mit einem einzelnen Patienten und/oder Standort verknüpft werden bzw. diesem zugeordnet werden.

Die vorliegende Erfindung sieht einen Datentransfer zwischen medizinischen Geräten und Applikationen vor, beispielsweise zwei medizinischen Geräten, Pumpe1 und Pumpe 2, welche über einen Server miteinander kommunizieren. Bei bestimmten kurzen Unterbrechungen/Pausen des Verfahrens läuft das System weiter und bei langen Unterbrechungen/Pausen wird der Zuordnungsdurchlauf erneut initiiert.

### Kurzbeschreibung der Figur

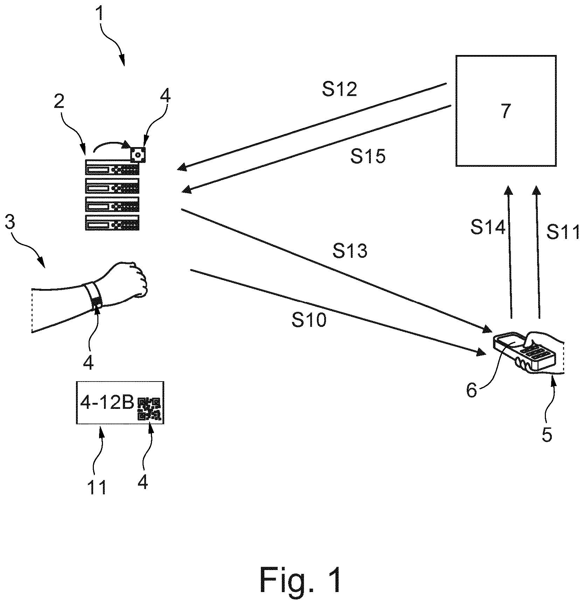

Fig. 1 ist eine schematische Darstellung zur Veranschaulichung eines Validierungssystems und eines Kontrollverfahrens gemäß der Ausführungsform der vorliegenden Offenbarung.

### Beschreibung der Ausführungsform

Nachstehend wird eine bevorzugte Ausführungsform der vorliegenden Offenbarung auf der Basis der zugehörigen Figur beschrieben. Die Figur ist lediglich von schematischer Natur und dient dem Verständnis der Erfindung. Die gleichen Elemente sind mit denselben Bezugszeichen bezeichnet.

Fig. 1 ist eine Darstellung zur Veranschaulichung eines Validierungssystems 1 gemäß der Ausführungsform der vorliegenden Offenbarung. Die in Fig. 1 dargestellten Pfeile mit den Bezeichnungen S10 bis S15 stellen Kommunikationsverbindungen/Datenpfade dar, welche in der nachfolgenden Beschreibung als Schritte S10 bis S15 bezeichnet sind. Das Validierungssystem 1 gemäß Fig. 1 hat unter anderem ein medizinisches Gerät 2. Das medizinische Gerät 2 ist in Fig. 1 beispielhaft dargestellt durch mehrere Infusionspumpen. Das medizinische Gerät 2 und ein dargestellter Patient (Patientenarm) 3 bzw. ein dargestelltes Patientenbett 11 haben jeweils eine Identifikationsmarkierung 4 als weiterer Teil des Validierungssystems 1, ausgebildet als QR-Code. Das medizinische Gerät 2 ist im Betrieb mit dem Patienten 3 verbunden (nicht dargestellt) oder befindet sich in der Nähe des Patientenbetts.

Des Weiteren hat das Validierungssystem 1 eine Identifizierungsvorrichtung 5. Die Identifizierungsvorrichtung 5 ist derart ausgebildet, um die Identifikationsmarkierungen 4 von dem medizinischen Gerät 2 und dem Patienten 3 und/oder dem Patientenbett 11 auszulesen. Die Identifizierungsvorrichtung 5 liest aus den Identifikationsmarkierungen 4 eine Geräte-ID, eine Patienten-ID und/oder eine Standort-ID/Patientenbett-ID aus. Die Identifizierungsvorrichtung 5 weist einen Display 6 auf, welcher dazu ausgebildet ist, Informationen und/oder Symbole anzuzeigen/zu visualisieren.

In Fig. 1 ist zudem ein Server 7 dargestellt, welcher sowohl mit dem medizinischen Gerät 2 als auch mit der Identifizierungsvorrichtung 5 kommuniziert.

Gemäß einem Zuordnungsdurchlauf liest die Identifizierungsvorrichtung 5 die Identifikationsmarkierungen 4 des medizinischen Geräts 2 und des Patienten 3 und/oder des Patientenbettes gemäß Schritt S10 aus. Die ausgelesene Geräte-ID, Patienten-ID und/oder Standort-ID werden als Datenpaket in einem Schritt S11 an den Server 7 übermittelt/gesendet.

Die nachfolgenden Schritte werden gemäß einem Überprüfungsdurchlauf durchgeführt. Dabei generiert der Server 7 in Schritt S12 aus der empfangenen Geräte-ID, Patienten-ID und/oder Standort-ID eine erste Transaktions-ID 8 und sendet diese erste Transaktions-ID 8 an das medizinische Gerät 2. Das medizinische Gerät 2 prüft, ob die dem medizinischen Gerät 2 entsprechende Geräte-ID in der empfangenen ersten Transaktions-ID 8 enthalten ist.

Sofern das der Fall ist, zeigt das Display des medizinischen Geräts 2 gemäß Schritt S13 eine modifizierte zweite Transaktions-ID 9 an. Die Modifikation gewährleistet, dass die Transaktions-ID 8 über das medizinische Gerät 2 geleitet wurde. Diese modifizierte zweite Transaktions-ID 9 wird von der Identifizierungsvorrichtung 5 eingelesen.

In Schritt S14 wird die zweite Transaktions-ID 9 an den Server 7 zurückgesendet und mit der im Server 7 gespeicherten ersten Transaktions-ID 8 verglichen. Bei einem positiven Vergleich der zweiten Transaktions-ID 9 mit der ersten Transaktions-ID 8, ist die Identifizierungsvorrichtung 5 dazu ausgebildet, die positive Zuordnung des medizinischen Geräts 2 zu dem Patienten 3 und/oder dem Patientenbett 11 gemäß dem Standort in einem optischen Symbol auf dem Display 6 zu visualisieren und die Zuordnung als gültig zu bezeichnen.

In einem Schritt S15 modifiziert der Server 7 die zweite Transaktions-ID 9 in eine dritte Transaktions-ID 10 und sendet die dritte Transaktions-ID 10 an das medizinische Gerät 2. Das medizinische Gerät 2 vergleicht die empfangene dritte Transaktions-ID 10 mit der gespeicherten zweiten Transaktions-ID 9. Bei einem positiven Vergleich der dritten Transaktions-ID 10 mit der zweiten Transaktions-ID 9, ist die Identifizierungsvorrichtung 5 dazu ausgebildet, die positive Zuordnung des medizinischen Geräts 2 zu dem Patienten 3 und/oder dem Patientenbett 11 gemäß dem Standort in einem optischen Symbol auf dem Bildschirm/Display des medizinischen Geräts 2 zu visualisieren und die Zuordnung als gültig zu bezeichnen.

Darüber hinaus ist es bevorzugt, wenn in einem weiteren Schritt S16 die positive Zuordnung bzw. Validierung zusätzlich auf dem Display der Identifizierungsvorrichtung 5 angezeigt wird. Diese Information der positiven Validierung wird über die Identifizierungsvorrichtung 5 beispielsweise auf optische, akustische oder haptische/vibrierende Weise an den Anwender/Nutzer rückgemeldet.

Nach der Durchführung dieser vorstehenden Schritte S10 bis S15 bzw. S16 enthalten alle von dem medizinischen Gerät/Produkt 2 gesendeten Daten, Patienten- und/oder Standortinformationen, die es ermöglichen eine Vielzahl von medizinischen Geräten 2 zu kombinieren und das medizinische Gerät 2 ist zudem dazu vorgesehen, eine unabhängige Validierung von eingehenden Kontrollaufträgen durchzuführen.

Es ist insbesondere vorgesehen, dass der Zuordnungsdurchlauf unabhängig von dem Überprüfungsdurchlauf durchgeführt werden kann.

### Bezugszeichenliste

- 1.: Validierungssystem
- 2.: medizinisches Gerät
- 3.: Patient
- 4.: Identifikationsmarkierung
- 5.: Identifizierungsvorrichtung
- 6.: Display
- 7.: Server
- 8.: erste Transaktions-ID
- 9.: zweite Transaktions-ID
- 10.: dritte Transaktions-ID

## Patentansprüche

1. Validierungssystem (1), mit
zumindest einem medizinischen Gerät (2), welches an einen Patienten (3) angeschlossen ist und sowohl das medizinische Gerät (2) als auch der Patient (3) und/oder ein Patientenbett mit jeweils zumindest einer Identifizierungsmarkierung (4) versehen ist,
einer Identifizierungsvorrichtung (5), welche mit einem Display (6) versehen ist und dazu vorgesehen und ausgebildet ist, die Identifikationsmarkierungen (4) auszulesen, und
einem Server (7), der mit dem zumindest einen medizinischen Gerät (2) und der Identifizierungsvorrichtung (5) kommuniziert und dazu vorgesehen und ausgebildet ist, das zumindest eine medizinische Gerät (2) einem entsprechenden Patienten (3) gemäß einem Zuordnungsdurchlauf zuzuordnen,
**dadurch gekennzeichnet, dass**
ein Überprüfungsmittel dazu vorgesehen und ausgebildet ist, in einem zu dem Zuordnungsdurchlauf unabhängigen Überprüfungsdurchlauf mittels einer von dem Server (7) generierten ersten Transaktions-ID (8), die sich aus einer Geräte-ID und einer Patienten-ID und/oder einer Standort-ID zusammensetzt, die Zuordnung des zumindest einen medizinischen Geräts (2) zu dem entsprechenden Patienten (3) zu überprüfen, und
das medizinische Gerät (2) in dem Überprüfungsdurchlauf dazu vorgesehen und ausgebildet ist, die erste Transaktions-ID (8) zu empfangen und eine geänderte zweite Transaktions-ID (9), wenn die erste Transaktions-ID (8) die Geräte-ID aufweist, an den Server (7) zu senden, und
der Server (7) in dem Überprüfungsdurchlauf dazu vorgesehen und ausgebildet ist, die empfangene zweite Transaktions-ID (9) mit der generierten und im Server (7) gespeicherten ersten Transaktions-ID (8) zu vergleichen.

2. Validierungssystem (1) gemäß Anspruch 1, **dadurch gekennzeichnet, dass** die Identifizierungsmarkierung (4) als ein QR-Code oder eine Identifikationsnummer ausgebildet ist und zumindest eine Geräte-ID und eine Patienten-ID und/oder eine Standort-ID aufweist.

3. Validierungssystem (1) gemäß Anspruch 1, **dadurch gekennzeichnet, dass** bei einem positiven Vergleich, die Identifizierungsvorrichtung (5) in dem Überprüfungsdurchlauf dazu vorgesehen und ausgebildet ist, die positive Zuordnung des medizinischen Geräts (2) zu dem Patienten (3) und/oder zu dem Ort in einem vorzugsweise optischen Symbol auf dem Display (6) zu visualisieren.

4. Validierungssystem (1) gemäß Anspruch 1, **dadurch gekennzeichnet, dass** der Server (7) in dem Überprüfungsdurchlauf dazu vorgesehen und ausgebildet ist, die zweite Transaktions-ID (9) in eine dritte Transaktions-ID (10) zu ändern und an das medizinische Gerät (2) zu senden.

5. Validierungssystem (1) gemäß Anspruch 4, **dadurch gekennzeichnet, dass** das medizinische Gerät (2) in dem Überprüfungsdurchlauf dazu vorgesehen und ausgebildet ist, die empfangene dritte Transaktions-ID (10) mit der gespeicherten zweiten Transaktions-ID (9) zu vergleichen.

6. Validierungssystem (1) gemäß Anspruch 5, **dadurch gekennzeichnet, dass** bei einem positiven Vergleich in dem medizinischen Gerät (2), die Identifizierungsvorrichtung (5) und/oder das medizinische Gerät (2) in dem Überprüfungsdurchlauf dazu vorgesehen und ausgebildet ist, die positive Zuordnung des zumindest einen medizinischen Geräts (2) zu dem Patienten (3) und/oder zu dem Ort in einem vorzugsweise optischen Symbol auf dem Display des medizinischen Geräts (2) und/oder der Identifizierungsvorrichtung (5) zu visualisieren.

7. Validierungssystem (1) gemäß einem der vorhergehenden Ansprüche, **dadurch gekennzeichnet, dass** das Validierungssystem (1) dazu vorgesehen und ausgebildet ist, die Patienten- und/oder Standortinformationen von einer Vielzahl von Medizingeräten/-produkten (2), welche einem einzigen Patienten (3) oder Ort zugeordnet sind, zu gruppieren.

8. Kontrollverfahren zur Sicherstellung, dass zumindest eine Patienten- und/oder Standortinformation zumindest einem medizinischen Gerät (2) zugeordnet und die Zuordnung überprüft wird, mit den folgenden Schritten:
Auslesen (S10) einer Identifizierungsmarkierung (4) und Ausgeben einer Geräte-ID, Patienten-ID und/oder Standort-ID als ein Datenpaket durch eine Identifizierungsvorrichtung (5),
Senden (S11) des Datenpakets an einen Server (7),
Generieren (S12) einer ersten Transaktions-ID (8) aus der Geräte-ID, Patienten-ID und/oder Standort-ID und Senden der generierten ersten Transaktions-ID (8) an das medizinische Gerät (2),
Ändern der ersten Transaktions-ID (8) in eine zweite Transaktions-ID (9), wenn die erste Transaktions-ID (8) die Geräte-ID aufweist,
Senden (S14) der zweiten Transaktions-ID (9) an den Server (7) und
Vergleichen der am Server (7) empfangenen zweiten Transaktions-ID (9) mit der im Server (7) gespeicherten ersten Transaktions-ID (8).

9. Kontrollverfahren gemäß Anspruch 8, das ferner folgenden Schritten aufweist:
Ändern (S15) der zweiten Transaktions-ID (9) in eine dritte Transaktions-ID (10) in dem Server (7) und Senden der dritten Transaktions-ID (10) an das medizinische Gerät (2) und Vergleichen der empfangenen dritten Transaktions-ID (10) mit der zweiten Transaktions-ID (9), und
Visualisieren und Anzeigen eines optischen Symbols auf dem Display des medizinischen Geräts (2) bei übereinstimmendem Vergleich.

## Claims

1. A validation system (1), with
at least one medical device (2) which is connected to a patient (3) and both the medical device (2) and the patient (3) and/or a patient's bed are each provided with at least one identification marker (4),
an identification device (5) which is provided with a display (6) and is provided and configured to read out the identification markers (4), and
a server (7) which communicates with the at least one medical device (2) and the identification device (5) and is provided and configured to assign the at least one medical device (2) to a corresponding patient (3) according to an assignment process,
**characterized in that**
a reviewing means is provided and configured to review the assignment of the at least one medical device (2) to the corresponding patient (3) in a reviewing process independent of the assignment process via a first transaction ID (8), composed of a device ID and a patient ID, and/or a location ID and generated by the server (7), and
the medical device (2) in the reviewing process is provided and configured to receive the first transaction ID (8) and to send a modified second transaction ID (9) to the server (7) if the first transaction ID (8) comprises the device ID, and
the server (7) in the reviewing process is provided and configured to compare the received second transaction ID (9) with the generated first transaction ID (8) stored in the server (7).

2. The validation system (1) according to claim 1, **characterized in that** the identification marker (4) is configured as a QR code or an identification number and comprises at least a device ID and a patient ID, and/or a location ID.

3. The validation system (1) according to claim 1, **characterized in that** in the event of a positive comparison, the identification device (5) in the reviewing process is provided and configured to visualize the positive assignment of the medical device (2) to the patient (3) and/or to the location in a preferably optical symbol on the display (6).

4. The validation system (1) according to claim 1, **characterized in that** the server (7) in the reviewing process is provided and configured to change the second transaction ID (9) to a third transaction ID (10) and to send it to the medical device (2).

5. The validation system (1) according to claim 4, **characterized in that** the medical device (2) in the reviewing process is provided and configured to compare the received third transaction ID (10) with the stored second transaction ID (9).

6. The validation system (1) according to claim 5, **characterized in that** in the event of a positive comparison in the medical device (2), the identification device (5) and/or the medical device (2) in the reviewing process is provided and configured to visualize the positive assignment of the at least one medical device (2) to the patient (3) and/or to the location in a preferably optical symbol on the display of the medical device (2) and/or the identification device (5).

7. The validation system (1) according to one of the preceding claims, **characterized in that** the validation system (1) is provided and configured to group the patient information and/or location information from a plurality of medical devices/products (2) associated with a single patient (3) or location.

8. A controlling method for ensuring that at least one piece of patient information and/or location information is assigned to at least one medical device (2) and that the assignment is reviewed, comprising the following steps:
reading (S10) an identification marker (4) and outputting a device ID, patient ID, and/or location ID as a data packet by an identification device (5),
sending (S11) the data packet to a server (7),
generating (S12) a first transaction ID (8) from the device ID, patient ID, and/or location ID, and sending the generated first transaction ID (8) to the medical device (2),
changing the first transaction ID (8) to a second transaction ID (9) if the first transaction ID (8) has the device ID,
sending (S14) the second transaction ID (9) to the server (7) and comparing the second transaction ID (9) received at the server (7) with the first transaction ID (8) stored in the server (7).

9. The controlling method according to claim 8, comprising the further steps of:
changing (S15) the second transaction ID (9) to a third transaction ID (10) in the server (7) and sending the third transaction ID (10) to the medical device (2) and comparing the received third transaction ID (10) with the second transaction ID (9), and
visualizing and displaying an optical symbol on the display of the medical device (2) if the comparison matches.

## Revendications

1. Système de validation (1), avec
au moins un appareil médical (2) qui est raccordé à un patient (3) et tant l'appareil médical (2) que le patient (3) et/ou un lit de patient sont dotés respectivement d'au moins un marquage d'identification (4),
un dispositif d'identification (5) qui est doté d'un écran (6) et est prévu et conçu pour lire les marquages d'identification (4), et
un serveur (7) qui communique avec le au moins un appareil médical (2) et le dispositif d'identification (5) et est prévu et conçu pour attribuer le au moins un appareil médical (2) à un patient (3) correspondant selon un passage d'attribution,
**caractérisé en ce que**
un moyen de vérification est prévu et conçu pour vérifier, dans un passage de vérification indépendant du passage d'attribution, l'attribution de l'au moins un appareil médical (2) au patient (3) correspondant au moyen d'un premier ID de transaction (8) généré par le serveur (7) qui se compose d'un ID d'appareil et d'un ID de patient et/ou d'un ID de site, et
l'appareil médical (2) est prévu et conçu pour recevoir, dans le passage de vérification, le premier ID de transaction (8) et envoyer un deuxième ID de transaction modifiée (9) au serveur (7) lorsque le premier ID de transaction (8) présente l'ID d'appareil, et
le serveur (7) est prévu et conçu pour comparer, dans le passage de vérification, le deuxième ID de transaction (9) reçu au premier ID de transaction (8) généré et enregistré sur le serveur (7).

2. Système de validation (1) selon la revendication 1, **caractérisé en ce que** le marquage d'identification (4) est conçu comme un code QR ou un numéro d'identification et présente au moins un ID d'appareil et un ID de patient et/ou un ID de site.

3. Système de validation (1) selon la revendication 1, **caractérisé en ce que,** en cas de comparaison positive, le dispositif d'identification (5) est prévu et conçu pour visualiser, lors du passage de vérification, l'attribution positive de l'appareil médical (2) au patient (3) et/ou au site dans un symbole de préférence optique sur l'écran (6).

4. Système de validation (1) selon la revendication 1, **caractérisé en ce que** le serveur (7) est prévu et conçu pour changer, lors du passage de vérification, le deuxième ID de transaction (9) en un troisième ID de transaction (10) et l'envoyer à l'appareil médical (2).

5. Système de validation (1) selon la revendication 4, **caractérisé en ce que** l'appareil médical (2) est prévu et conçu pour comparer, lors du passage de vérification, le troisième ID de transaction (10) reçu au deuxième ID de transaction (9) enregistré.

6. Système de validation (1) selon la revendication 5, **caractérisé en ce que,** en cas de comparaison positive dans l'appareil médical (2), le dispositif d'identification (5) et/ou l'appareil médical (2) est prévu et conçu pour visualiser, lors du passage de vérification, l'attribution positive de l'au moins un appareil médical (2) au patient (3) et/ou au site dans un symbole de préférence optique sur l'écran de l'appareil médical (2) et/ou du dispositif d'identification (5).

7. Système de validation (1) selon l'une quelconque des revendications précédentes, **caractérisé en ce que** le système de validation (1) est prévu et conçu pour grouper les informations de patient et/ou de site d'une pluralité d'appareils/produits médicaux (2) qui sont attribués à un seul patient (3) ou site.

8. Procédé de contrôle pour garantir qu'au moins une information de patient et/ou de site est attribuée à au moins un appareil médical (2) et que l'attribution est vérifiée, avec les étapes suivantes :
lecture (S10) d'un marquage d'identification (4) et émission d'un ID d'appareil, d'un ID de patient et/ou d'un ID de site comme un paquet de données par l'intermédiaire d'un dispositif d'identification (5),
envoi (S11) du paquet de données à un serveur (7),
génération (S12) d'un premier ID de transaction (8) à partir de l'ID d'appareil, de l'ID de patient et/ou de l'ID de site et envoi du premier ID de transaction (8) généré à l'appareil médical (2),
changement du premier ID de transaction (8) en un deuxième ID de transaction (9) lorsque le premier ID de transaction (8) présente l'ID d'appareil,
envoi (S14) du deuxième ID de transaction (9) au serveur (7) ; et
comparaison du deuxième ID de transaction (9) reçu au niveau du serveur (7) au premier ID de transaction (8) enregistré sur le serveur (7).

9. Procédé de contrôle selon la revendication 8, qui présente en outre les étapes suivantes :
changement (S15) du deuxième ID de transaction (9) en un troisième ID de transaction (10) sur le serveur (7) et envoi du troisième ID de transaction (10) à l'appareil médical (2) et comparaison du troisième ID de transaction (10) reçu au deuxième ID de transaction (9), et
visualisation et affichage d'un symbole optique sur l'écran de l'appareil médical (2) en cas de comparaison concordante.
